# EUROPEAN PATENT SPECIFICATION

(11) **EP 2 297 306 B1**
(45) Date of publication and mention of the grant of the patent: **14.09.2016**
(21) Application number: 09758732.3
(22) Date of filing: 01.06.2009
(51) Int. Cl.: A61K 39/00, C12N 5/078, A61K 35/12

(54) **HUMAN FACILITATING CELLS**
MENSCHLICHE FC-ZELLEN
CELLULES FACILITANTES HUMAINES

(30) Priority: 30.05.2008 US 57724 P
(43) Date of publication of application: 23.03.2011
(73) Proprietor: University Of Louisville Research Foundation, Inc., Louisville, KY 40202 (US)
(72) Inventor: ILDSTAD, Suzanne, T., Prospect KY 40059 (US); ELLIOTT, Mary, Jane, Brandenburg KY 40108 (US)
(74) Representative: Gowshall, Jonathan Vallance
(86) International application number: PCT/US2009/003340
(87) International publication number: WO 2009/148568

(56) References cited:
- WO-A1-99/26639
- WO-A2-2005/023982
- US-A- 5 772 994
- US-A1- 2004 185 043
- US-A1- 2004 228 845
- US-A1- 2005 118 142
- US-A1- 2007 098 693
- US-A1- 2007 098 693
- GANGOPADHYAY ET AL: "Bone marrow-derived CD8alpha<+>TCR<-> cells that facilitate allogeneic bone marrow transplantation are a mixed population of lymphoid and myeloid progenitors", EXPERIMENTAL HEMATOLOGY, ELSEVIER INC, US, vol. 35, no. 12, 17 October 2007 (2007-10-17), pages 1847-1857, XP022356324, ISSN: 0301-472X, DOI: 10.1016/J.EXPHEM.2007.07.016
- SCHUCHERT M J ET AL: "CHARACTERIZATION OF A NEWLLY DISCOVERED T-CELL RECEPTOR BETA-CHAIN HETERODIMER EXPRESSED ON A CD8+ BONE MARROW SUBPOPULATION THAT PROMOTES ALLOGENEIC STEM CELL ENGRAFTMENT", NATURE MEDICINE, NATURE PUBLISHING GROUP, NEW YORK, NY, US, vol. 6, no. 8, 1 August 2000 (2000-08-01), pages 904-909, XP002936229, ISSN: 1078-8956, DOI: 10.1038/78667
- FUGIER-VIVIER ISABELLE J ET AL: "Plasmacytoid precursor dendritic cells facilitate allogeneic hematopoietic stem cell engraftment", JOURNAL OF EXPERIMENTAL MEDICINE, vol. 201, no. 3, 7 February 2005 (2005-02-07), pages 373-383, XP9157387, ISSN: 0022-1007
- GRIMES H L ET AL: "Graft facilitating cells are derived from hematopoietic stem cells and functionally require CD3, but are distinct from T lymphocytes", EXPERIMENTAL HEMATOLOGY, ELSEVIER INC, US, vol. 32, no. 10, 1 October 2004 (2004-10-01), pages 946-954, XP004613163, ISSN: 0301-472X, DOI: 10.1016/J.EXPHEM.2004.07.011

## Description

### TECHNICAL FIELD

This invention relates to human facilitating cells, and the use of such cells in therapeutic protocols.

### BACKGROUND

Facilitating cells (FCs) from mouse have been described. See, for example, U.S. Patent No. 5,772,994. FCs are not stem cells, but significantly improve the initial and long-term engraftment of stem cells. For example, transplantation of stem cells alone only prolongs survival of a transplant patient, while the presence of FCs result in sustained and long-term HSC engraftment. See, also, Kaufman et al., 2005, J. Exp. Med., 201:373-83).

US 2007/098693 discloses mammalian hematopoietic stem cells and CD8+/TCR-LFCs.

### SUMMARY

The present invention is defined in the claims.

The present invention relates to human facilitating cells (hFCs), methods of isolating hFCs, and hFCs for facilitating reconstitution of a damaged or destroyed hematopoietic system with stem cells as well as for inducing donor-specific tolerance for the transplantation of donor cells, tissues and solid organs.

In one aspect, a cellular composition is provided that includes human hematopoietic stem cells (HSCs), wherein the HSCs have a phenotype of CD34+; and human facilitating cells (hFCs), wherein the hFCs have a phenotype of CD8+ /TCR- / CD56^{dim/neg}.In one embodiment, the hFCs CD56^{dim/neg} phenotype is CD56^{dim}.

According to this disclosure, hFCs further can have a phenotype of CD3⁺ / CD16⁺ / CD 19+ / CD52+. In addition, hFCs can have a phenotype, without limitation, of CXCR4, CD123, HLADR, NKp30, NKp44, NKp46, CD11c, and CD 162, and hFCs further can be characterized by the presence of markers such as, without limitation, CD11a, CD11b, CD62L, and FoxP3. Typically, the hFCs described herein improve the engraftment ability of the HSCs compared to HSCs engrafted in the absence of the hFCs. Cellular compositions as described herein can include at least about 50% (e.g., 75%, or 90%) of the hFCs. Typically, the phenotype of the cells are determined by antibody staining or flow cytometry.

In one aspect, a pharmaceutical composition is provided that includes a cellular composition as described herein. In another aspect, methods of treating a human suffering from a disease are provided. Such methods generally include administering a pharmaceutical composition that includes a cellular composition as described herein to a human. In still another aspect, methods of transplanting donor cells, tissues, or organs into a human recipient are provided. Such methods generally include administering a pharmaceutical composition that includes a cellular composition as described herein to the human recipient.

Such methods can further include partially conditioning the human by exposure to total body irradiation, an immunosuppressive agent, a cytoreduction agent, or combinations thereof prior to the administration of the pharmaceutical composition. In one embodiment, the total body irradiation is 200 cGy. A pharmaceutical composition as described herein can be administered intravenously.

In one embodiment, the disease is an autoimmune disease such as diabetes, multiple sclerosis, or systemic lupus erythematosus. In another embodiment, the disease is an infection by an immunodeficiency virus or hepatitis. In yet another embodiment, the disease is chosen from a hematopoietic malignancy, anemia, hemoglobinopathies, and an enzyme deficiency.

Representative donor tissues or organs include, without limitation, heart, skin, liver, lung, heart and lung, kidney, pancreas, or an endocrine organ such as a thyroid gland, parathyroid gland, a thymus, adrenal cortex, or adrenal medulla. Donor cells can be pancreatic islet cells, neurons, or myocytes.

In yet another aspect, methods for obtaining a cellular composition that includes at least about 30% hFCs is provided. Such a method generally includes providing a hematopoietic cell composition; and removing cells from the
hematopoietic cell composition that have a phenotype of alpha beta TCR- and gamma delta TCR-. Such methods can further include selecting for cells that have a phenotype of CD8+; selecting for cells that have a phenotype of CD56^{dim/neg}; and selecting for cells that have a phenotype of CD3+ / CD16+ / CD19+ / CD52+. In addition, such methods can further include selecting for cells that have a phenotype of CXCR4, CD 123, HLADR, NKp30, NKp44, NKp46, CD11c, and CD 162, and such methods can still further include selecting for cells that have a phenotype of, wiithout limitation, CD11a, CD11b, CD62L, and FoxP3. A cellular composition produced by such methods also can include CD34+ hematopoietic stem cells (HSCs).

Cells can be removed and/or selected for using an antibody (e.g., an antibody conjugated to a magnetic bead). In addition or alternatively, the hematopoietic cell composition can be separated by density gradient centrifugation to obtain cells in the mononuclear cell fraction. In one embodiment, the hematopoietic cell composition is contacted with a growth factor. The hematopoietic cell composition can be obtained from bone marrow, thymus, peripheral blood, fetal liver, or embryonic yolk sac. In one embodiment, cellular composition includes at least about 50% hFCs (e.g., at least about 75% hFCs).

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting.

The details of one or more embodiments of the invention are set forth in the accompanying drawings and the description below. Other features, objects, and advantages of the invention will be apparent from the drawings and detailed description, and from the claims.

### DESCRIPTION OF DRAWINGS

Figure 1 shows the gating strategy for sorting and enumeration of human HSCs.
Figure 2 shows the gating strategy for sorting and enumerating human hFCs.
Figure 3 is a graph showing that hFCs significantly improved the colony-forming ability of HSCs following incubation with the HSCs compared to the combination of HSCs and hFCs with no incubation (i.e., direct plating).
Figure 4 is a graph showing the results of immunological monitoring in response to a number of stimulators 1-month post-transplantation in a transplant patient (SCD#3).
Figure 5 is a graph showing the absolute neutrophil count (ANC) in a transplant patient (SCD#4).
Figure 6 is a graph showing the chimerism of a transplant patient (SCD#3).
Figure 7 is a graph showing the source of hemoglobin from a transplant patient (SCD#3).
Figure 8 is a graph showing the chimerism of a transplant patient (SCD#4).
Figure 9 is a graph showing the source of hemoglobin from a transplant patient (SCD#4).
Figure 10 is a graph showing the reticulocyte counts for a transplant patient (SCD#4).
Figure 11 is a graph showing the ANC following solid organ transplant in a patient (#12).
Figure 12 is a graph showing the recovery of B cells, CD4 + cells, and CD8+ cells within 3 months following solid organ transplant in a patient (#12).
Figure 13 is a graph showing the platelet count following solid organ transplant in a patient (#12).
Figure 14 is a graph showing the amount of the ANC and white blood cells (WBCs) following solid organ transplant in a patent.
Figure 15 is a graph showing the platelet count following solid organ transplant in a patient.
Figure 16 is a graph showing the platelet count following solid organ transplant in a patient (#12).
Figure 17 is a graph showing the percent chimerism in a solid organ transplant patient.
Figure 18 is a graph showing the percent chimerism in a solid organ transplant patient (# 12).

### DETAILED DESCRIPTION

Human hFCs (hFCs) are provided that facilitate initial stem cell engraftment and that are required for sustained HSC engraftment. Also provided are methods of purifying such hFCs from bone marrow or other physiological sources of hematopoietic cells. A variety of separation procedures are provided, which generally are based on the presence or absence of specific markers as disclosed herein.

### Human Facilitating Cells (hFCs), Cellular Compositions Containing hFCs, and Methods of Making

Human facilitating cells (hFCs) have been identified and are described herein. hFCs are generally characterized as CD8+, alpha beta TCR-, gamma delta TCR-, and CD56^{dim/neg}. As indicated herein, hFcs typically lack TCR cells, and typically include cells that do not express CD56 (CD56^{neg}) as well as cells that express a relatively small amount of CD56 (CD56^{dim}). hFCs can include, however, cells that express a relatively large amount of CD56 (CDS6^{bright}). hFCs also can be characterized by the presence of markers including, but not limited to, CD3, CD16, CD19, CD52, CXCR4, CD123, HLADR, NKp30, NKp44, NKp46, CD11c, and CD162. hFCs further can be characterized by the presence of markers such as, without limitation, CD11a, CD11b, CD62L, and FoxP3. hFCs can be obtained from bone marrow, or any other physiologic source of hematopoietic cells such as, without limitation, the spleen, thymus, blood, embryonic yolk sac, or fetal liver.

Once a source of hematopoietic cells is obtained, hFCs can be purified (or substantially purified) by various methods that typically use antibodies that specifically bind particular markers to select those cells possessing (or lacking) those particular markers. Cell separation techniques include, for example, flow cytometry using a fluorescence activated cell sorter (FACS) and specific fluorochromes; biotin-avidin or biotin-streptavidin separations using biotin conjugated to cell surface marker-specific antibodies and avidin or streptavidin bound to a solid support (e.g., affinity column matrix or a plastic surface); magnetic separations using antibody-coated magnetic beads; or destructive separations such as antibody and complement or antibody bound to cytotoxins or radioactive isotopes. Methods of making antibodies that can be used in cell separations are well known in the art. See, for example, U.S. Patent No. 6,013,519.

Separation using antibodies directed toward specific markers can be based upon negative or positive selections. In separations based on negative selection, antibodies that are specific for markers that are present on undesired cells (non-hFCs) and that are not present on the desired cells (hFCs) are used. Those (undesired) cells bound by the antibody are removed or lysed and the unbound cells retained. In separations based on positive selection, antibodies that are specific for markers that are present on the desired cells (hFCs) are used. Those cells bound by the antibody are retained. It will be understood that positive and negative selection separations may be used concurrently or sequentially. It will also be understood that the present disclosure encompasses any separation technique that can be used to enrich or purify the hFCs described herein.

One well-known technique for antibody-based separation is flow cytometry using, for example, a FACS. Briefly, separation by flow cytometry can be performed as follows. A suspended mixture of hematopoietic cells are centrifuged and resuspended in media. Antibodies that are conjugated to fluorochromes are added to allow the binding of the antibodies to the specific cell surface markers. The cell mixture is then washed and run through a FACS, which separates the cells based on their fluorescence, which is dictated by the specific antibody-marker binding. Separation techniques other than flow cytometry additionally or alternatively can be used to obtain hFCs. One such method is biotin-avidin (or streptavidin)-based separation using affinity chromatography. Typically, such a technique is performed by incubating hematopoietic cells with biotin-coupled antibodies that bind to specific markers, followed by passage of the cells through an avidin column. Biotin-antibody-cell complexes bind to the column via the biotin-avidin interaction, while non-complexed cells pass through. The column-bound cells can be released by perturbation or other known methods. The specificity of the biotin-avidin system is well-suited for rapid separation.

Flow cytometry and biotin-avidin techniques provide highly specific means for cell separation. If desired, less specific separations can be utilized to remove portions of non-hFCs from the hematopoietic cell source. For example, magnetic bead separations can be used to initially remove non-facilitating differentiated hematopoietic cell populations including, but not limited to, T-cells, B-cells, natural killer (NK) cells, and macrophages (MAC) as well as minor cell populations including megakaryocytes, mast cells, eosinophils, and basophils. In addition, cells can be separated using density-gradient separation. Briefly, hematopoietic cells can be placed in a density gradient prepared with, for example, Ficoll or Percoll or Eurocollins media. The separation can then be performed by centrifugation or automatically with, for example, a Cobel & Cell Separator '2991 (Cobev, Lakewood, CO). Additional separation procedures may be desirable depending on the source of the hematopoietic cell mixture and its content. For example, if blood is used as a source of hematopoietic cells, it may be desirable to lyse red blood cells prior to the separation of any fraction.

Although separations based on specific markers are disclosed, it will be understood that the present disclosure encompasses any separation technique(s) that result in a cellular composition that is enriched for hFCs, whether that separation is a negative separation, a positive separation, or a combination of negative and positive separations, and whether that separation uses cell sorting or some other technique, such as, for example, antibody plus complement treatment, column separations, panning, biotin-avidin technology, density gradient centrifugation, or other techniques known to those skilled in the art. Most sources of hematopoietic cells include about 0.5% - 8% hFCs. Therefore, the separations such as those disclosed herein can yield cellular compositions that are enriched for hFCs (i.e., include a greater number of hFCs than are found naturally in physiological hematopoietic cell sources). For example, cellular compositions are provided in which at least about 30% (e.g., at least about 40%, 50%, 60% or more) of the cells are hFCs as described herein. These compositions could be referred to as "enriched" for hFCs. Further separations can yield cellular compositions in which at least about 65% of the cells (e.g., at least about 70%, 75%, 80%, 85%, 90%, 95%, or 99%) are hFCs as described herein. These compositions that contain a higher percentage of hFCs could be referred to as "substantially purified" or "purified" for hFCs.

An exemplary method of obtaining a cellular composition that includes at least about 30% hFCs is described herein. In this example, bone marrow is the source of the hematopoietic cells. Bone marrow typically is harvested from the long bones (e.g., femora or tibia), but also can be obtained from other bone cavities or the spine. Bone marrow is harvested by various methods well known to those skilled in the art. In one embodiment, the HSCs are removed from the bone marrow using antibodies that bind CD34+. See, for example, U.S. Patent No. 5,061,620 or the LC Laboratory Cell Separation System, CD34 Kit (CellPro, Inc., Bothell, WA). One or more of the positive- and/or negative-selections described herein can be used to further enrich or purify the hFCs. In another embodiment, a number of non-hFCs and non-HSCs can be removed from the hematopoietic cells using one or more of the negative selections described herein. The resulting cellular composition contains HSCs, hFCs, and other immature progenitor cells such as immature lymphoid and myeloid progenitor cells. Further, T cells, also known as graft vs. host disease (GVHD)-producing cells, can be specifically removed from the cellular composition using antibodies directed toward T cell-specific markers such as CD3+ and alpha beta TCR+.

Furthermore, hFCs can be obtained via cell sorting. hFCs can be purified for CD8+, alpha beta TCR-, gamma delta TCR-, CD56^{dim/neg} as described herein.

### Methods of Using hFCs and Cellular Compositions Containing hFCs

The ability of hFCs to enhance engraftment of donor bone marrow cells in a recipient indicates that hFCs are useful in facilitating various therapy protocols. Using a cellular composition that is enriched for hFCs (i.e., contains at least about 30% hFCs) significantly improves durable engraftment and eliminates graft vs. host disease (GVHD). The use of hFCs in establishing a chimeric immune system can significantly expand the scope of diseases that can be treated using bone marrow transplantation. Although not bound by any particular mechanism, it is believed that, once administered, the hFCs home to various hematopoietic cell sites in the recipient's body, including bone cavity, spleen, fetal or adult liver, and thymus. The hFCs become seeded at the proper sites, engraft, and begin establishing a chimeric immune system. It is possible that both the stem cells and the hFCs complex together to seed the appropriate site for engraftment.

Generally, the methods of the present invention relate to the administration of cellular compositions comprising hFCs (e.g., a cellular composition having at least 30% hFCs) to a recipient in combination with donor stem cells and any additional donor bone marrow components desired (e.g., stem and/or progenitor cells such as HSCs). The donor bone marrow typically is depleted of T-cells. The hFCs can be autologous or syngeneic to the recipient, but allogeneic hFCs also can be used. It is noted that the hFCs and the HSCs generally are MHC-matched to one another, but neither the hFCs nor the HSCs need to be MHC-matched to the recipient. Typically, a chimeric immune system is one that is at least about 1% (e.g., at least about 2%, 3%, 4%, 5%, 6%, 7%, 8%, 9%, 10% or more) donor origin.

Cellular compositions that include hFCs are administered to the recipient. If the hFC and HSC cellular compositions are separate compositions, they can be administered simultaneously, or separately within a relatively close period of time. Alternatively, a hFC-enriched cellular composition (e.g., purified hFCs) can be combined and mixed with donor HSCs prior to administration. In humans, T-cell-depleted bone marrow can be administered in amounts between about 1 x 10⁸ cells and 3 x 10⁸ cells per kilogram of dosing weight of the recipient. In humans, the number of HSCs administered can be between about 1 x 10⁵ and 10 x 10⁶ HSCs per kg of recipient dosing weight. A similar range of hFCs can be administered. The numbers of cells that are used will depend on many factors, including the condition of the recipient's health. Cellular compositions generally are administered intravenously, but other modes of administration such as direct bone injection can be used.

Traditionally, methods of establishing a chimeric immune system required destroying the immune system of the recipient, which results in ablation of the recipient's HSCs. This may be accomplished by techniques well known to those skilled in the art and include, without limitation, lethally irradiating the recipient with selected levels of total body irradiation, administering specific toxins to the recipient, administering specific monoclonal antibodies attached to toxins or radioactive isotopes to the recipient, or combinations thereof. Notably, administering the hFCs described herein to a recipient significantly reduces the amount of immunosuppression required for engraftment. For example, destroying a recipient's immune system often involves lethally irradiating the recipient with 950 RADs (R; radiation absorbed dose) of total body irradiation (TBI), while the procedures described herein utilize as little as 25 centigray (cGy) to 200 cGy of TBI.

The ability to establish successful chimerism allows for significantly improved survival following transplant. The present invention provides for methods of transplanting a donor physiological component, such as, for example, organs, tissue, or cells. Using the hFCs in the methods disclosed herein results in a recipient who has a chimeric immune system, which is completely immunotolerant to transplanted donor organ, tissue, or cells, but competently rejects third party grafts. Transplanted donor organ, tissue, or cells are able to perform their respective functions in the recipient. For example, transplanted islet cells can provide an effective treatment for diabetes. In addition, permanent acceptance of endocrine tissue grafts (thyroid, parathyroid, adrenal cortex, adrenal medulla, islets) as well as kidney, liver, heart, and composite tissues such as face, hand and other extremities has been demonstrated. It will be understood that a mixed chimeric immune system can be produced in a recipient before, during, or after transplantation, but typically is produced before the transplantation.

Beyond transplantation, the ability to establish a successful chimeric hematopoietic system in a recipient can be used to treat other diseases or disorders that are not currently treated by bone marrow transplantation because of the morbidity and mortality associated with GHVD. Autoimmune diseases involve attack of an organ or tissue by one's own immune system. However, when a chimeric immune system is established, the body can relearn what is foreign and what is self. Establishing a chimeric immune system using the hFCs described herein can reduce or halt the autoimmune attack causing the condition. Autoimmune diseases that can be treated using the hFCs described herein include, for example, type I diabetes, systemic lupus erythematosus, multiple sclerosis, rheumatoid arthritis, psoriasis, or colitis. It may also be possible to treat Alzheimer's disease. The cellular compositions that include the hFCs disclosed herein also can be used to treat hemoglobinopathies such as, for example, sickle cell anemia, spherocytosis or thalassemia, as well as metabolic disorders such as Hunters disease, Hurlers disease, and enzyme defects.

In accordance with the present invention, there may be employed conventional molecular biology, cell biology, microbiology and biochemical techniques within the skill of the art. Such techniques are explained fully in the literature. The invention will be further described in the following examples, which do not limit the scope of the invention defined by the claims.

### EXAMPLES

### Section A-Colony Forming Cell Assays

### Example 1-Purification of HSC and hFC

HSC and hFC were isolated from Human Vertebral Bone Marrow (VBM) or Mobilized Peripheral Blood (MPB) by multiparameter, live sterile cell sorting (FACSVantage SE: Becton Dickinson). Briefly, VBM or MPB was stained with directly labeled monoclonal antibodies (mAbs) at saturating concentrations for 30 min. HSCs: CD34+ / CD45+; and hFCs: CD8+ / TCR- / CD56^{dim/neg}_{.} Both cell populations were sorted and analyzed for purity. Only 85% or greater purity levels were accepted.

### Example 2-HSC and hFC sorting and enumeration

HSCs were sorted and enumerated based on the ISHAGE protocol. See, Sutherland et al., 1996, "The ISHAGE guidelines for CD34+ cell determination by flow cytometry," J. Hematotherapy, 5:213-26. Briefly, CD45-FITC/CD34-PE combination parameters provided a clinically relevant reflection of the peripheral blood stem/progenitor cell compartment. Plot 1 was formatted with Forward Scatter (FSC; x-axis) vs Side Scatter (SSC; y-axis), and a region (R1) was drawn around the lymphocyte, monocyte and granulocyte populations excluding debris. From R1, Plot 2 was formatted with CD45 FITC versus Side Scatter, and R1 was drawn so that CD45- events were excluded. From R2, Plot 3 was formatted with CD34 PE versus Side Scatter, and R3 was drawn only around the CD34+ population. From R3, Plot 4 was formatted with CD45-FITC versus SSC of CD34+ cells. Cells forming a cluster with characteristic low SSC and low to intermediate CD45 fluorescence were gated and designated R4. Nonspecific stained events were excluded from this region. From R4, Plot 5 was formatted with FSC (x-axis) versus SSC (y-axis). A cluster of events meeting all the fluorescence and light scatter criteria of CD34+ stem/progenitor cells appeared in Plot 5.

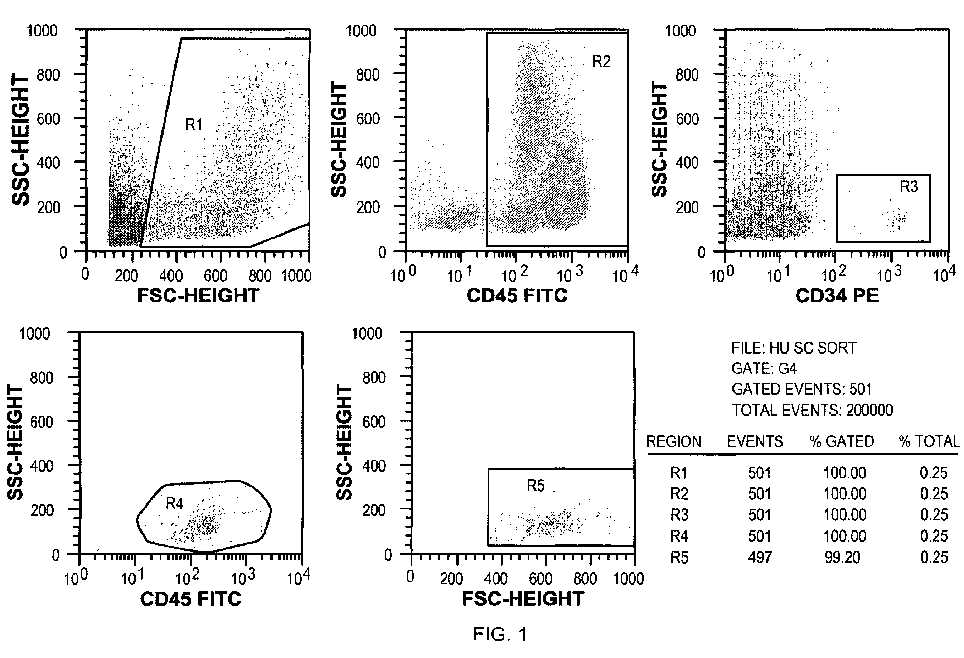

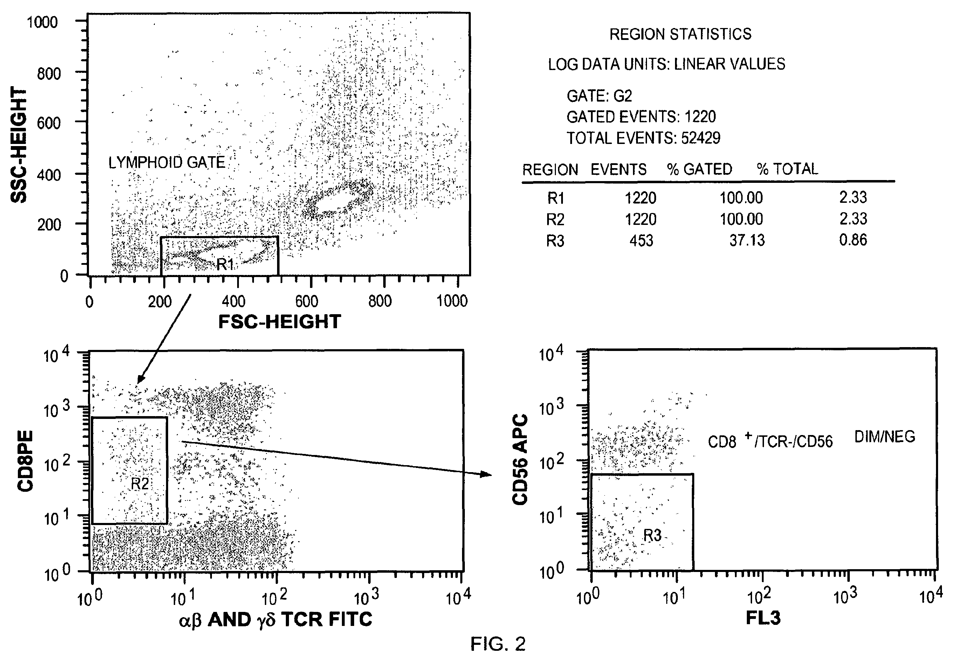

Figure 1 shows the gating strategy for the sorting and enumeration of human HSCs using the ISHAGE protocol. Figure 2 shows the gating strategy for the sorting and enumeration of human hFCs.

### Example 3-Colony-forming cell assay

Following cell sorting, HSCs (CD45+ / CD34+) alone or HSCs and hFCs (CD45+ / CD34+ plus CD8+ / TCR- / CD56^{dim/neg}) or HSCs + T cells as a control were either immediately plated in methylcellulose (0 hr) or pre-incubated for 18 hrs in cell culture media before plating in methylcellulose. All cell samples were cultured in quadruplicate. After 14 days of culturing at 37°C and 5% CO₂, colonies containing more than 50 cells were scored.

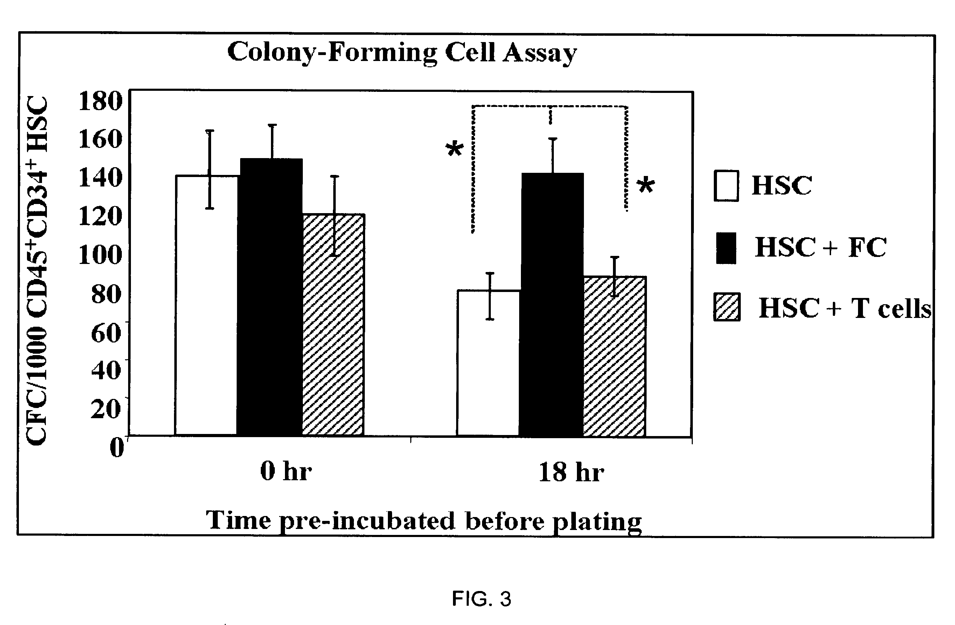

Without pre-incubation, there was no significant difference in colonies generated by HSC alone vs. HSC plus hFC. Strikingly, when HSCs were co-incubated with hFCs for 18 hrs prior to placement in the CFC assay, hFC significantly (p < 0.005) enhanced colony formation compared to HSC alone and HSC co-incubated with CD8+ T cells. Figure 3 shows the results of the CFC assay described herein. Results are expressed as CFC frequency per 1000 HSC plated. Data represents the mean ± SE of 10 different experiments. These results suggest that human hFCs, like mouse hFCs, exert a protective effect on HSCs and promote the generation of more primitive multipotent progenitors *in vitro.*

### Section B- Characterization of Human hFCs In Vivo

### Example 1- Chimerism and Engraftment in a Mouse Model

It has been shown previously that CD8⁺/TCR⁻ hFCs enhance engraftment of purified HSCs in allogeneic and syngeneic mouse recipients (Fugier et al., 2005, J. Exp. Med., 201(3):373-383). In addition, it has been shown in mice that hFCs enhance the clonogenicity and promote the generation of more primitive multipotent HSC progenitors *in vitro* (Rezzoug et al., 2008, J. Immunology, 180(1):49-57).

The first goal was to achieve human HSC chimerism in a mouse model. Briefly, CD34⁺, CD45⁺ human HSCs were sorted from G-CSF mobilized peripheral blood, and 100,000 sorted human HSCs were transplanted into NOD/SCID/IL2 receptor (IL2R) γ chain^{null} mice conditioned with 325 cGy TBI. Whole blood was collected from transplanted mice one month following transplantation, and PBL typing was performed using antibodies specific for human T cells, B cells, natural killer cells, dendritic cells, and monocytes. Results showed that an average of 3.2% human HSC chimerism was achieved following transplantation with 100,000 hHSCs.

Experiments then were performed in which 100,000 hHSCs alone or 100,000 hHSCs + 300,000 hFCs were transplanted into NOD/SCID/IL2Rγ^{null} mice conditioned with 325 cGy TBI. Multilineage PBL typing was performed at 30 days after transplantation as described above.

The results of these experiments demonstrated that the HSC + hFC group produced a higher percentage of human T cells (CD4, CD8, DC; see Table 1) and human monocytes (CD33; Table 2) compared to the HSC alone group. The percentage of donor chimerism in lymphoid gate and myeloid gate are summarized in Table 3.

**Table 1. Percentage of human T cells, NK cells, B cells, and DCs in lymphoid gate**

| Group | Mouse | T cells | | | | NK | B cell | DC |
|---|---|---|---|---|---|---|---|---|
| | | CD8 | CD4 | CD3 | αβ/δγ TCR | CD56 | CD19 | CD11c |
| HSC alone | A | 0 | 0.1 | 0.1 | 0.1 | 0.1 | 0.1 | 0 |
| | B | 0 | 0 | 0.1 | 0.3 | 0.2 | 0 | 0 |
| | C | 0 | 0 | 0 | 0.1 | 0 | 0 | 0 |
| HSC+hFC | D | 0.1 | 0.5 | 0.4 | 0.5 | 0.1 | 0.1 | 0.1 |
| | E | 0.1 | 0.1 | 0.1 | 0.1 | 0.1 | 0 | 0.1 |
| | F | 0 | 0.1 | 0.1 | 0.1 | 0.1 | 0.1 | 0.1 |

**Table 2. Percentage of human DCs and monocytes in myeloid gate**

| Group | Mouse | CD11c | CD33 |
|---|---|---|---|
| HSC alone | A | 4.8 | 9.2 |
| | B | 0.6 | 5 |
| | C | 0 | 0 |
| HSC+hFC | D | 3.2 | 6.6 |
| | E | 4.1 | 8.1 |
| | F | 8.2 | 14.9 |

**Table 3. Percentage of human hematopoietic cells**

| Group | Mouse | Lymphoid | Myeloid |
|---|---|---|---|
| | | CD45 | CD45 |
| HSC alone | A | 1.5 | 9.2 |
| | B | 1.5 | 5.8 |
| | C | 0.3 | 0.3 |
| HSC+hFC | D | 1.1 | 8.2 |
| | E | 0.8 | 9.4 |
| | F | 1.2 | 15.9 |

### Section C-Treatment of Sickle Cell Disease (SCD) in Humans

### Example 1-The sickle cell disease (SCD) preliminary experiment

Two sickle-cell disease (SCD) patients were previously treated in a pilot experiment to try to establish mixed chimerism. Both SCD patients were at high risk for complications from their disease. Whether a combination of 200 cGy TBI with fludarabine, MMF, and CyA could establish engraftment in patients with SCD was evaluated. Only transient engraftment, however, was achieved. The conditioning was well tolerated, and no severe adverse events occurred; however, endogenous hematopoiesis reappeared.

To overcome the transfusion/sensitization barrier, improvements were made to the protocol. For example, Campath, which is a humanized anti-CD52 monoclonal antibody that is a powerful lytic agent for mature T cells, B cells and NK cells, was added to the clinical conditioning regimen. Two cycles of Campath were administered (month -2 and month -1) with the rationale that the first cycle would deplete mature B cells and cause homeostatic proliferation of memory B cells to replace the depleted B cells and the second cycle would deplete the proliferating memory B cells. It was hypothesized that the broad lymphoid specificity of Campath would provide a powerful approach to target T and B cells in the recipient that mediate the alloreactivity induced by transfusion therapy.

In one example, four doses of 10 mg/day of Campath-1H were administered at day -53 to -50, and another four doses of 7 mg/day of Campath-1H were administered at day -24 to -21. 30 mg/m² of Fludarabine was administered at day -5 to -3, and 200 cGy total body irradiation was administered at day -1 along with Mycophenolate mofetil and cyclosporine, which was continued until durable engraftment. FCs + HSCs were transplanted at day 0.

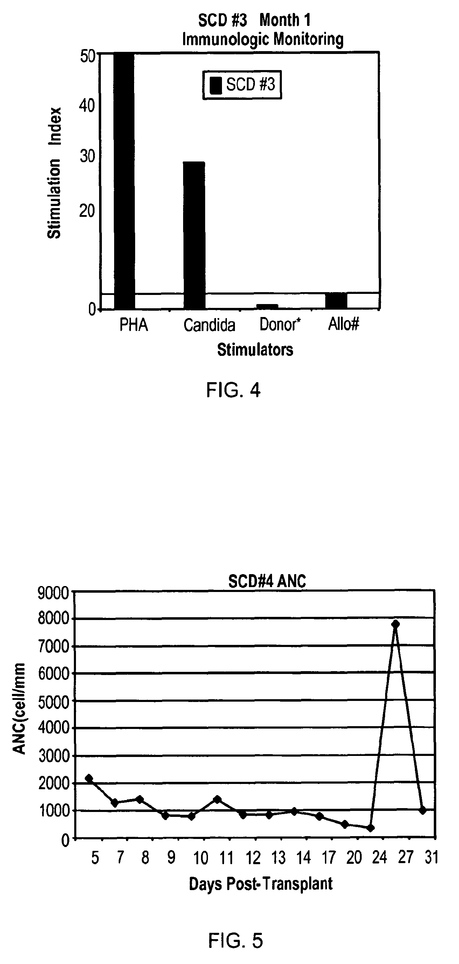

To date, two subjects with SCD have been successfully transplanted under the revised protocol. Both subjects have maintained engraftment at 27 and 24 months post-transplant and are asymptomatic and transfusion independent. It was demonstrated that mixed chimerism can be established with minimal toxicity in sensitized recipients through partial recipient conditioning followed by transplantation with HSCs and hFCs to reduce the risk of GVHD while preserving engraftment. The reduced-intensity conditioning approach described herein is safe, well-tolerated and, in combination with the HSC + hFC graft, sufficient to induce stable mixed chimerism and dominantly normal RBC production in transfused patients. Immunocompetence to respond to PHA, *Candida,* and alloantigen returned by 1 month post-transplant (Figure 4; a stimulation index of >3 is positive (horizontal line on graph)). The nadir occurred between day 9 and day 24 for both patients (absolute neutrophil count [ANC] < 1,000) (Figure 5).

### Example 2-SCD Patient #3 - Transplanted in Nov 2005

SCD#3 (Date of Birth 2-11-98) is a African American female who experienced multiple pain crises and episodes of acute chest syndrome. She was maintained on transfusion therapy. Her HLA-identical sister with sickle cell trait served as her donor. The patient was conditioned with four doses of Campath-1H (10 mg/day) starting at day -53, and a second round of four doses of Campath-1H (7 mg/day) starting at day -24. She received 3 doses of fludarabine starting at day -4, and then 200 cGy of TBI on day 0.

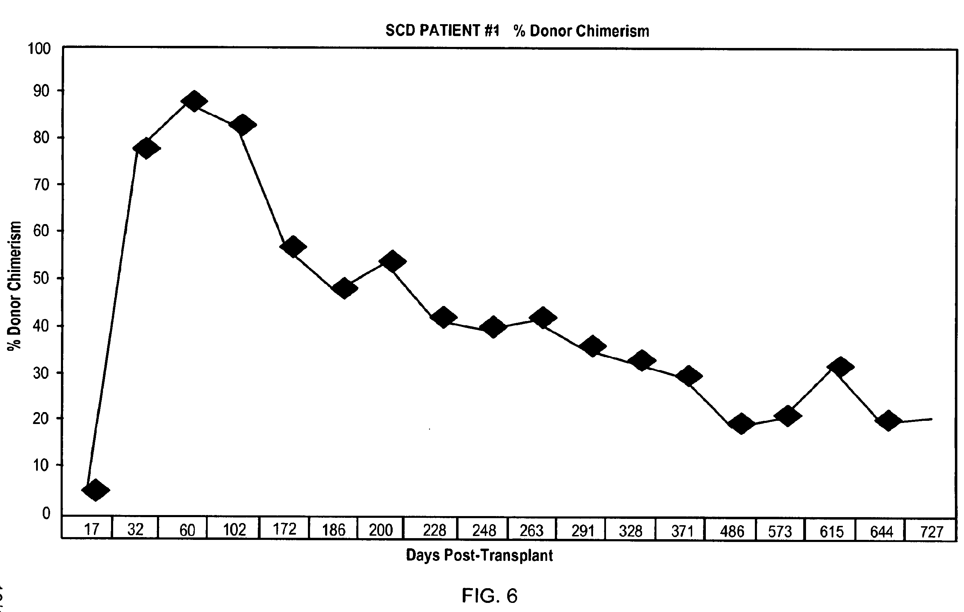

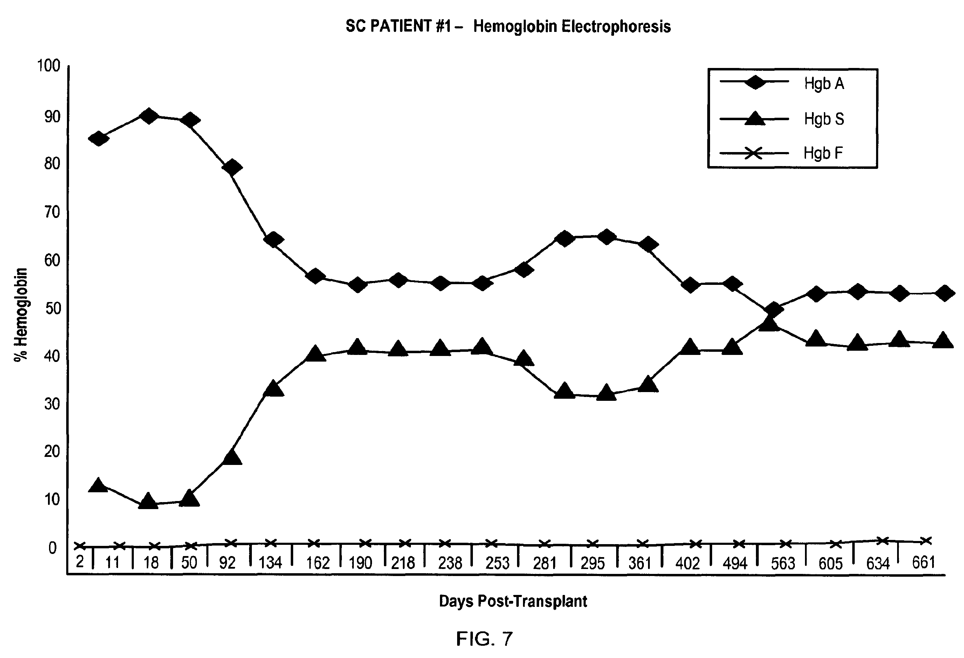

Post-transplant, she was treated with cyclosporine and MMF for 22 months. The immunosuppression was subsequently tapered and has been discontinued for more than 18 months. The patient received 14.5 x 10⁶/kg CD34 cells, 43.5 x 10⁶/kg alpha beta TCR cells and 6 x 10⁶/kg hFCs. She showed 5% donor cell chimerism on Day 17 and 78% donor cell chimerism on day 32. She has been asymptomatic and has not required transfusions post transplant. As of day 727, she was 21% donor cell chimeric (Figure 6), and she has no evidence of GVHD. Although her total donor chimerism was approximately 30%, she was producing nearly 100% donor-derived trait RBC (Figure 7).

During the processing procedure for SCD #3, some difficulty was experienced in recovering the correct fraction in the cell separation (Percoll) procedure due to the density of SCD trait marrow cells. Because the donor/recipient pair were HLA matched, the decision was made to abort the process and not deplete the product. Therefore, the patient received whole bone marrow. However, the efficacy of the conditioning was established in this candidate.

### Example 3-SCD Patient #4 - Transplanted in March 2006

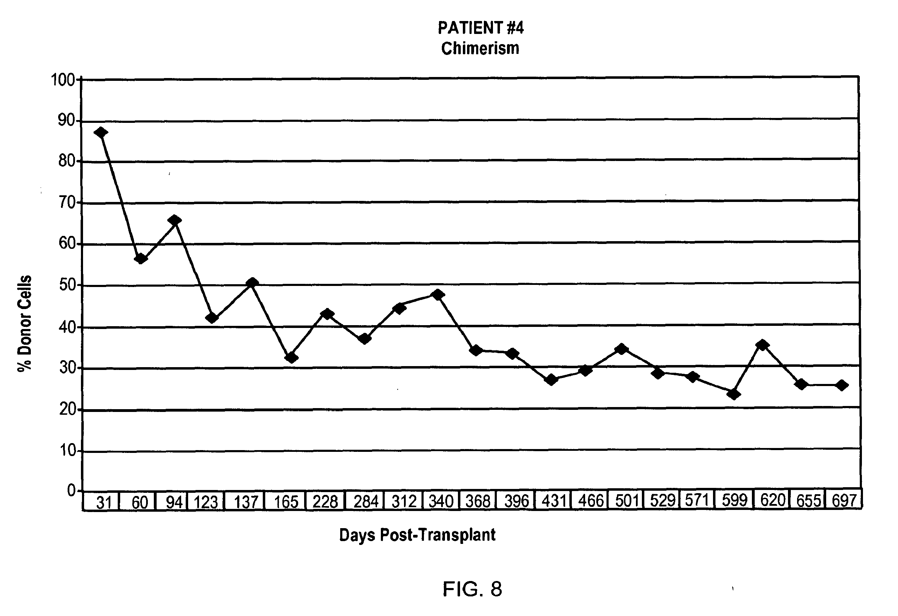

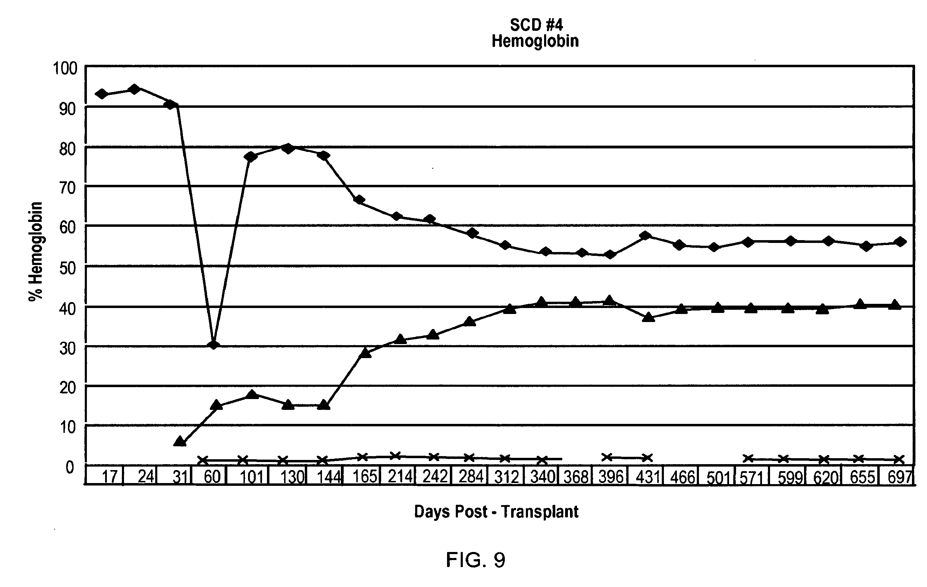

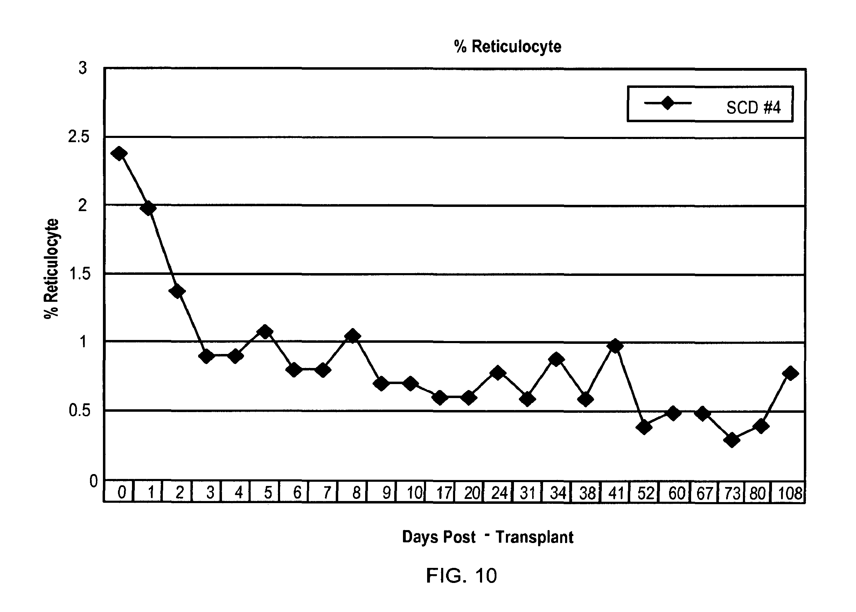

SCD#4 (Date of birth 5/23/96) is a Nigerian male who suffered multiple pain crises and two acute chest syndromes prior to starting red cell exchange in 1999. The patient's HLA-identical sibling who had sickle cell trait served as his donor. The patient received the same non-myeloablative conditioning as SCD#3. His HSC + hFC dose was 5.24 x 10⁶/kg CD34 cells, 0.55 x 10⁶/kg αβ-TCR cells and 0.35 x 10⁶/kg hFC. He tolerated the conditioning very well and engrafted and chimeric (88% donor cells) at one month based on FISH. His donor chimerism was 28% as of day 697 (Figure 8). Donor T cell chimerism was 34% at day 501. The patient has remained asymptomatic since his transplant and is producing predominantly normal RBC (Figure 9). The reticulocyte counts for patient SCD#4 has ranged between 0.5% and 1%, which is within normal ranges (Figure 10).

### Example summary

This section described successful transplantation of two heavily-transfused SCD patients using HLA-identical marrow from sibling donors. Both patients were successfully transplanted using reduced-intensity non-myeloablative conditioning and have remained disease free for > 2 years. At enrollment, they were transfusion-dependent and at very high risk for painful crises and other complications. Both patients have been successfully weaned from immunosuppression.

### Section D-Prevention of Graft vs. Host Disease (GVHD) Following Solid Organ Transplant.

### Example 1-Patient recruitment

Candidates for the protocol were selected from the list of patients awaiting renal transplantation or who were being evaluated for transplantation. This selection process was carried out by the transplant surgeons and the transplant nurse coordinators of the University of Louisville Institute of Cellular Therapeutics ("the Institute").

### Example 2-Inclusion Criteria

A candidate patient must be between the ages of 18 and 65 years and meet the Institution's criteria for renal transplantation for end-organ failure. A candidate patient must be receiving his or her first renal transplant. A candidate patient must be receiving a renal transplant only. The crossmatch must be negative between the donor and the recipient. Women who are of child bearing potential must have a negative pregnancy test (urine test is acceptable) within 48 hours prior to initiating TBI and must agree to use reliable contraception for 1 year following transplant. Candidate patients must exhibit no evidence of donor-specific antibody, presently or historically.

### Example 3-Exclusion Criteria

Patients are not candidates if they have a clinically active bacterial, fungal, viral or parasitic infection, or if they are pregnant. Patients are not eligible if they exhibit clinical or serologic evidence of viral infection that would preclude the recipient from receiving a kidney transplant. A patient is not a candidate if they have received previous radiation therapy at a dose which would preclude TBI, if there is a positive crossmatch between the donor and the recipient, or if there is evidence for immunologic memory against the donor. Patients also are excluded if their body mass index (BMI) is less than 18 or greater than 35.

### Example 4-Donor Selection Criteria

Donors for this protocol must meet all of the Institute's criteria for renal and stem cell transplant.

### Example 5-Protocol

The timing for all manipulations is relative to the TBI conditioning of the recipient on day 0. Beginning day -3 and for up to four days, 10 µg/kg G-CSF was administered b.i.d. Collection began on day 0. On day 0, a CD34 count was performed prior to giving the final dose of G-CSF. HSC + hFC transplantation was scheduled 4 to 6 weeks prior to the desired date of kidney harvest. The donation and transplant of the kidney is not schedules until the donor's platelet counts have returned to baseline and safe levels for kidney donation (e.g., greater than 100,000/µl of whole blood).

The visits for G-CSF administration, blood stem cell donations, and blood draws are summarized in Table 4. The 'X' marks what will occur on each visit.

**Table 4. Donor Mobilization**

| Visits | Symptom Assessment | Filgrastim / G-CSF | Blood Stem Cell Donation | Blood Draws |
|---|---|---|---|---|
| Screening | X | | | X |
| Preparation, Day -3 | X | X | | X |
| Preparation, Day -2 | X | X | | |
| Preparation, Day -1 | X | X | | |
| Preparation, Day 0, First donation | X | X | X | X |
| 2 Days after donation | X | | | |
| 1 Week after donation | X | | | |
| Potential blood draws to test for donor chimerism in the recipient (up to three years) | | | | X |

### Example 6-Pre-transplant conditioning

Cell dose (HSC + hFC) as well as degree and type of conditioning of the recipient were independent variables that influence engraftment. In the current protocol, cell dose and conditioning were optimized until > 1% donor chimerism was established. The initial target cell dose for HSC + hFC was ≥ 1 x 10⁸ CD34+/kg. The first patients received 200 cGy TBI, fludarabine (30 mg/m2 day -3 to -1), and post-transplant immunosuppression with MMF (15 mg/kg q 12 h beginning day 0), and FK506 (0.02 mg/kg q 12 h beginning day -1) for six months, or as clinical need required. The decision to use either FK506 (tacrolimus) or cyclosporine was left to the physician because patients differ in their ability to tolerate either drug. The marrow was infused on day +1. The schedule is shown in Table 5.

**Table 5**

| Day | Treatment | Dose |
|---|---|---|
| -3 | Fludarabine after dialysis (if required) | 30 mg/m² |
| -2 | Fludarabine after dialysis (if required) | 30 mg/m² |
| -1 | Fludarabine | 30 mg/m² |
| 0 | Start MMF and FK506 or cyclosporine | |
| 0 | TBI (200 cGy) | 35-40 cGy/min |
| | Harvest donor marrow and process to obtain HSC + hFC | |
| +1 | Infuse HSC + hFC after dialysis (if required) | |
| +28-60 | Renal transplantation; continue MMF and calcineurin inhibitors | |

If the recipient required dialysis, the dosing of fludarabine and the HSC + hFC infusion occurred after dialysis on the specified days. On the morning of HSC + hFC infusion, an extra liter of volume was removed from dialysis to account for the volume of HSC + hFC. Dialysis then was scheduled for 48 hrs or later following HSC + hFC infusion to give the cells an optimum opportunity to home to the marrow compartment.

### Example 7-Outcomes

A minimum of 30 days post-HSC + hFC infusion or as long as the recipient needs to fully recover from the stem cell transplant procedure passed before the renal transplant was performed from the same donor. The following algorithm was used based on the outcome of the HSC + hFC transplant.
1) if the recipient exhibited chimerism of ≥ 1% and was determined to be tolerant to the donor, at least six months of Prograf and MMF was administered while donor:host tolerance to the kidney is established. The recipient did not receive Campath-1H or any additional immunosuppression at the time of transplant.
2) if the recipient did not engraft, the patient as tested by flow crossmatch prior to transplant to ensure no donor-specific antibodies developed. If no donor-specific antibody was present, the patient underwent living donor kidney transplant using conventional lymphodepletion induction with Campath-1H followed by maintenance immunosuppression with FK506 and MMF. Other lymphodepletion approaches for induction therapy such as ALG can be used in place of Campath per standard of care.
3) if donor-specific antibody developed, the patient was assessed and a clinically appropriate antibody reduction protocol implemented prior to transplantation. Patient sensitization was not expected.

### Example 8-Cell dosing algorithm

The goal of the study was to engineer a graft with adequate HSCs, hFCs and progenitors for allogeneic engraftment while avoiding GVHD. A cell dosing algorithm was established that is tied to the maximum allowable alpha beta T cell dose. For example, if toxicity (GVHD) did not occur but engraftment was not durable, the maximum allowable T cell dose was increased by 1 unit (see below). The maximal allowable T cell dose containing as many HSCs, hFCs, and progenitors was administered. The algorithm that was used is shown in Table 6.

**Table 6**

| | |
|---|---|
| HSC, hFC, progenitors | As many as possible |
| alpha beta TCR+ T cells | For HLA matched, there is no cap. For HLA mismatched, the maximal allowable is determined by the last safe dose in the kidney, heart, liver tolerance, sickle cell, and MS protocol |
| NK cells, B cells | Record and report doses |
| gamma delta TCR+ T cells | Record and report doses |

The present cell dosing currently allows a maximum of 3.0 x 10⁶ alpha beta T cells/kg recipient body weight, which was the starting dose. Each patient was followed for at least 28 days. If no evidence of engraftment was observed, the maximal allowable alpha beta-TCR dose was increased by one unit (4 x 10⁵/kg recipient body weight). The maximal allowable alpha beta-TCR dose was increased in subjects until stable engraftment was achieved without significant GVHD. For HLA-matched transplants, there was no maximum T cell cap and cell dose was not increased based on the outcome of the matched transplants. For patients who are mismatched, the maximal allowable alpha beta-TCR dose was determined.

If significant (>0.5%) donor engraftment was observed in the first 28 days, the subject was followed for an additional 28 days to assess the incidence of acute GVHD before the cell dose was increased. It was expected that the majority of cases of acute GVHD would be apparent by 8 weeks post-transplant. If evidence of severe GVHD was seen, the maximal allowable T cell dose was reduced to a level proven safe. To date, GVHD has not been observed.

### Example 9-HSCs + hFCs

Donor PBMC was processed to enrich for hFCs and HSC. Approximately 85% of total bone marrow composition was removed, including GVHD-producing T-cells and B-cells, using a ferromagnetic approach. The resultant product was enriched for hFCs, HSCs and progenitors. After the adequacy of the processing was confirmed by flow cytometry, the HSC + hFC graft was approved for infusion. A delay in the bone marrow transplantation of up to 72 hours following donor cell harvest was accepted to allow for bone marrow processing and transplantation. Dose-adjusted Bactrim and Valcyte (if CMV+) or Valtrex (if CMV-) prophylaxis was started. The patient was carefully monitored during and after infusion of marrow to detect any changes in, for example, respiration, blood pressure, or angioedema, which may be an indication of hypersensitivity.

### Example 10-Post-transplant immunosuppression

Subjects enrolled in this protocol received standard immunosuppression at the discretion of the attending physician and according to institutional protocol. For deceased donor kidney/HSC + hFC recipients and living donor HSC + hFC recipients without demonstratable donor chimerism at 1 month, this generally included Prograf plus MMF after lymphodepletion induction therapy with ALG or Campath. Prograf levels were maintained between 8-12 ng/ml. Starting on day 0, MMF was generally dosed at 1-1.5 gms b.i.d. A one time dose of Campath, 30 mg HIV, was optionally given in the operating room. SoluMedrol was given at a dose of 500 mg IV in the operating room one hour prior to the Campath dose, then post-operatively on day 1 at a dose of 250 mg IV and on post op day 2 at a dose of 125 mg. FK506 and MMF were continued for at least 6 months in patients who were chimeric to promote engraftment and tolerance induction.

### Example 11-Preliminary solid organ transplant protocol

Preliminary experiments demonstrated the success and safety of HSCs + hFCs and immune-based nonmyeloablative conditioning in renal transplant recipients. Dosing of the HSCs + hFCs was performed to maximize safety and optimize HSC and hFC content. The overall goal was to completely avoid GVHD. Solid organ tolerance protocols were begun with a maximum of 0.2 x 10⁶ T cells/kg recipient body weight to perform a dose-escalation. Total alpha beta T cells were used in the dose-escalation experiments since the other effector cells for GVHD (NK, B-cells, and APC) are all present in amounts proportional to total alpha beta T cells. CD34 and hFC dose were optimized within the maximum allowable T cell dose. No significant immunologic events (i.e., rejection episodes or antibody production) were observed in any of the patients. None of the patients developed GVHD, but only transient chimerism was observed.

Since September 2004, nine heart and eleven kidney patients have been transplanted using the dose-escalation strategy set forth in Table 7. Patients 5, 6, 12, and 13, which are highlighted in Table 7, are described in more detail; three patients underwent simultaneous kidney/HSC + hFC transplantation from living donors and the remaining patient received his kidney from a deceased donor. All four patients were conditioned with 200 cGy TBI and underwent lymphodepletion induction therapy with Campath followed by maintenance immunosuppression with MMF and a calcineurin inhibitor. They did not receive fludarabine. The 4 patients are briefly described below.

Patient #5 is a 55-year-old male who underwent a deceased donor renal allograft/HSC + hFC transplant in Sept 2005. The patient received 3.7 x 10⁶ CD34 and 0.8 x 10⁶ hFC per kg recipient body weight. The conditioning was well tolerated and no adverse events related to the approach occurred. The donor and recipient shared a 1/6 HLA-antigen match. The patient experienced the anticipated nadir, and then recovered immune function and endogenous hematopoiesis. He is doing well with a recent serum creatinine of 2.1.

Patient #6 is a 58-year-old male who underwent a living donor kidney/HSC + hFC transplant from an unrelated friend in Nov 2005. The patient received 1.33 x 10⁶ CD34 and 0.18 x 10⁶ hFC per kg recipient body weight. The conditioning was well tolerated and no adverse events related to the approach occurred. The donor and recipient shared a 1/6 HLA-antigen match. The patient experienced the anticipated nadir, and then recovered immune function and endogenous hematopoiesis. He is doing well, with a recent serum creatinine of 2.0.

Patient #12 is a 37-year-old female who underwent a living donor kidney/HSC + hFC transplant from her cousin in Oct 2007. She received 2.24 x 10⁶ CD34 and 0.41 x 10⁶ hFC per kg recipient body weight. The conditioning was well tolerated and no adverse events related to the approach occurred. The donor and recipient shared a 2/6 HLA-antigen match. The patient experienced the anticipated nadir, and then recovered immune function and endogenous hematopoiesis. She is doing well with a recent creatinine of 1.2.

Patient #13 is a 49-year-old female who underwent a kidney/HSC + hFC transplant from her brother in Nov 2007. The patient received 3.85 x 10⁶ CD34 and 0.78 10⁶ hFC per kg recipient body weight. The conditioning was well tolerated and no adverse events related to the approach occurred. The donor and recipient shared a 4/6 HLA-antigen match. The patient experienced the anticipated nadir, and then recovered immune function and endogenous hematopoiesis. She is doing well with a recent creatinine of 1.2.

**Table 7. Cell dosing for patients (per kg recipient weight)**

| # | Transplant | Date of Transplant | Source | TBI (cGy) | T cells* | CD34* | hFC* |
|---|---|---|---|---|---|---|---|
| 1 | Heart | Sept 2004 | VB | 200 | 0.2 | 1.99 | 0.23 |
| 2 | Kidney | Oct 2004 | MPB | 200 | 0.2 | 0.78 | 0.02 |
| 3 | Heart | Dec 2004 | VB | 200 | 0.4 | 5.70 | 1.07 |
| 4 | Kidney | Feb 2005 | VB | 200 | 0.6 | 3.80 | 0.14 |
| 5 | Kidney | Sept 2005 | VB | 200 | 0.8 | 3.70 | 0.80 |
| 6 | Kidney | Nov 2005 | MPB | 200 | 1.0 | 1.33 | 0.18 |
| 7 | Heart | March 2006 | VB | 200 | 1.2 | 0.71 | 0.08 |
| 8 | Heart | March 2006 | VB | 200 | 1.2 | 5.60 | 0.74 |
| 9 | Heart | May 2006 | VB | 200 | 1.4 | 4.69 | 0.55 |
| 10 | Heart | Feb 2007 | VB | 200 | 1.8 | 3.81 | 0.72 |
| 11 | Heart | Aug 2007 | VB | 200 | 1.8 | 2.08 | 0.75 |
| 12 | Kidney | Oct 2007 | MPB | 200 | 2.2 | 2.24 | 0.41 |
| 13 | Kidney | Nov 2007 | MPB | 200 | 2.2 | 3.85 | 0.78 |
| 14 | Heart | Jan 2008 | VB | 200 | 2.6 | 2.67 | 1.61 |
| 15 | Kidney | March 2008 | MPB | 200 | 3.0 | 9.26 | 9.34 |
| 16 | Kidney | April 2008 | MPB | 200 | 3.0 | 1.45 | 5.81 |
| 17 | Heart | April 2008 | VB | 200 | 3.4 | 4.86 | 1.87 |
| 18 | Kidney | Feb 2009 | IC | 200 | 0.96 | 0.90 | 0.16 |
| 19 | Kidney | April 2009 | MPB | 200 | 3.8 | 2.53 | 4.48 |
| 20 | Kidney | May 2009 | MPB | 200 | 3.8 | 3.60 | 0.90 |

| | | | | | | | |
|---|---|---|---|---|---|---|---|
| * x 10⁶ cells; VB = vertebral body; MPB = mobilized peripheral blood; IC = iliac crest | | | | | | | |

### Example 12-Results of preliminary protocol

Initially, the chimerism observed was low (< 0.2%) and only transient. However, as the total cell dose was increased, durable mixed chimerism was achieved. The immune response to the graft was modulated by the marrow infusion as evidenced by transient donor-specific tolerance in mixed lymphocyte reaction (MLR) assays observed in the more recently transplanted patients and the absence of any clinical or histologic rejection episodes.

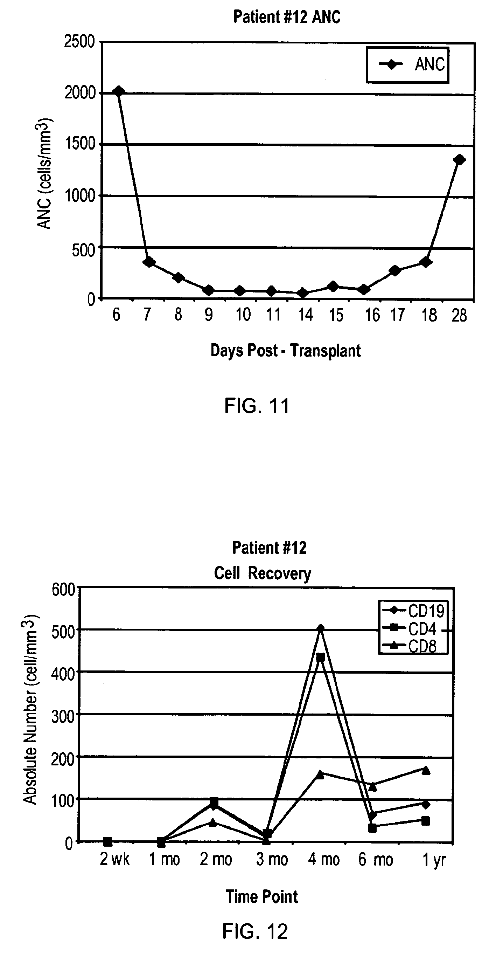

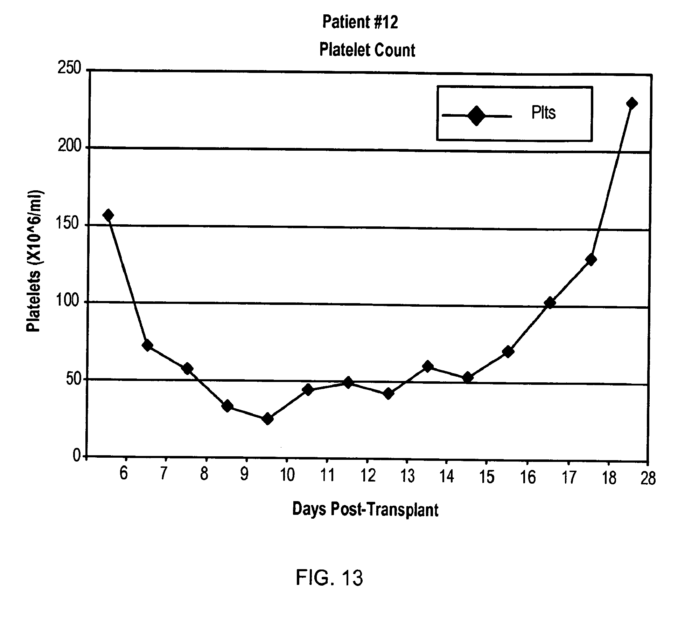

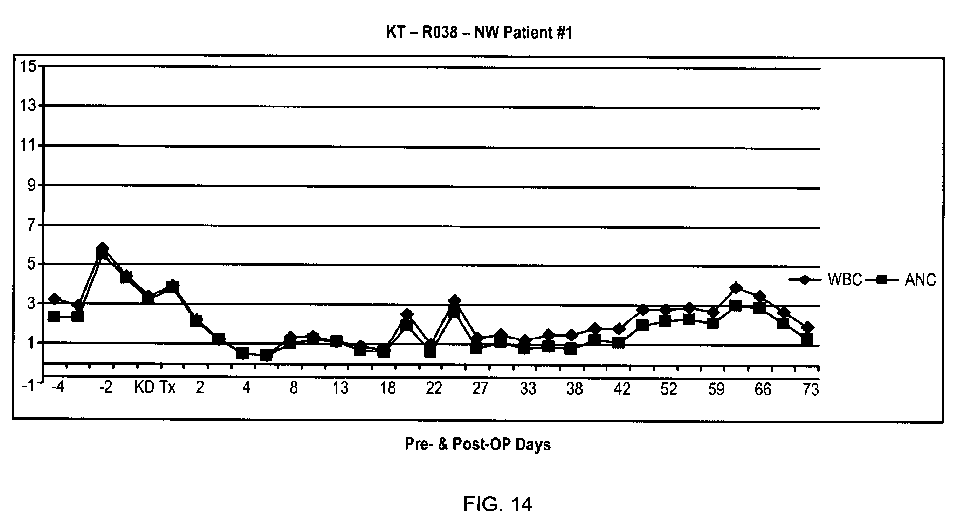

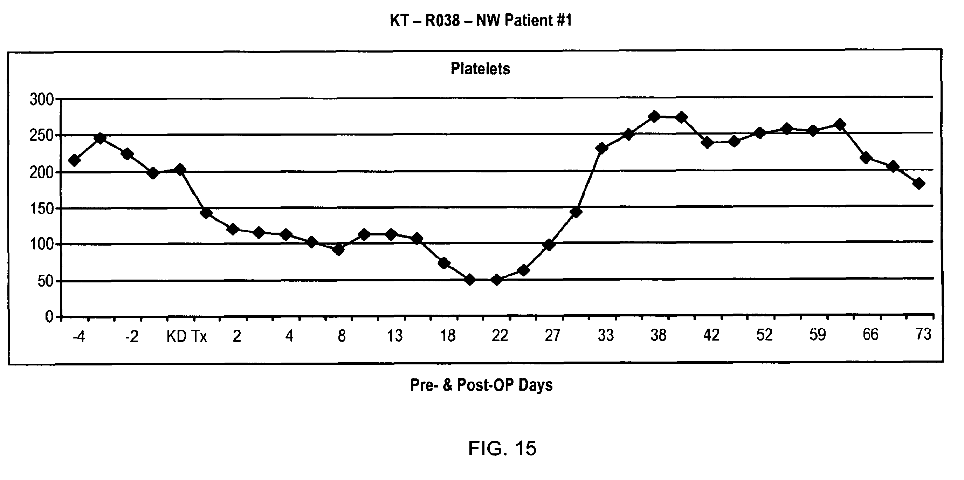

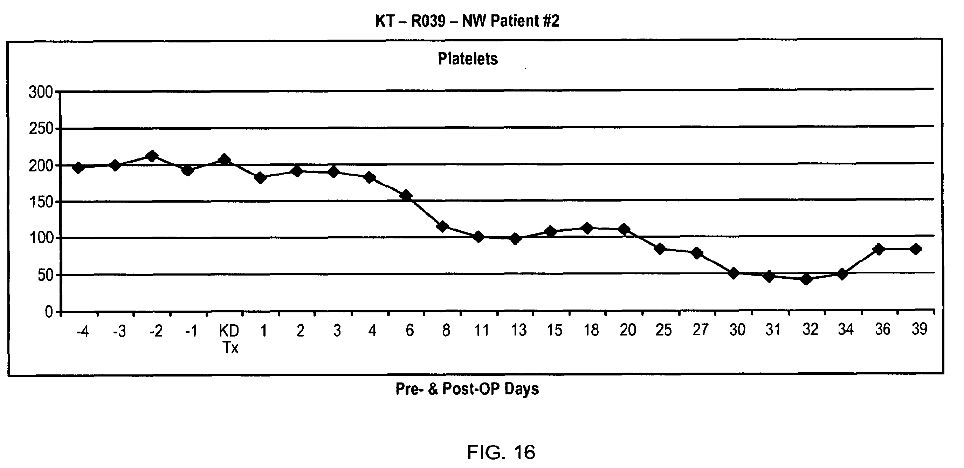

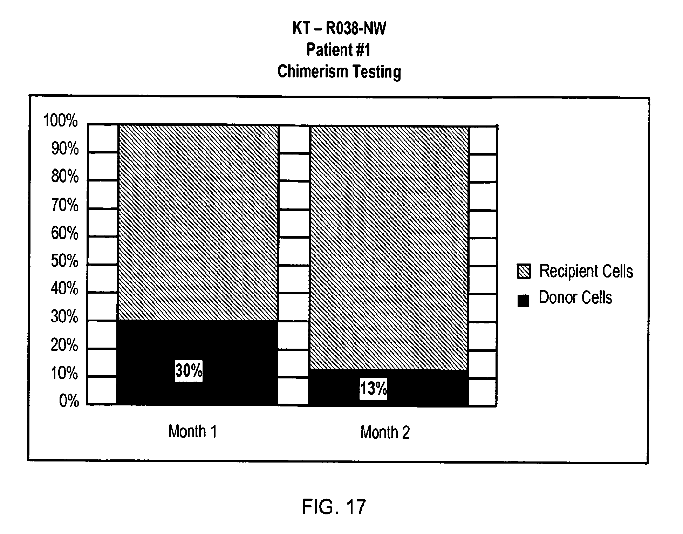

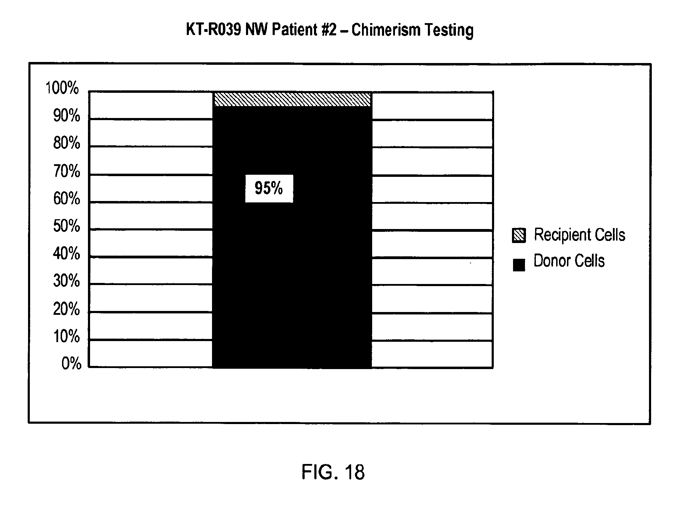

The fact that endogenous hematopoiesis resumed in those patients who did not engraft confirmed the non-myeloablative nature of the conditioning. There was an expected nadir of absolute neutrophil count (ANC) of less than 1,000 that occurred in the recipients between 7 and 18 days (Figures 11 and 14), which was managed as an outpatient in Patient #12. It was found that administration of G-CSF did not accelerate recovery. The MMF and FK506 was continued through the nadir to promote engraftment. Recovery of B cells (CD19), CD4+ cells, and CD8+ cells occurred by 3 months in the Campath-lymphodepleted recipient #12 (Figure 12). Platelet counts were determined following solid organ transplant (Figures 13, 15 and 16). The platelet nadir, if present, typically was brief and usually did not require transfusion therapy. The chimerism that was established in solid transplant patients is shown in Figures 17 and 18.

### Example 13-Modified protocol for solid organ transplant

The kidney/HSC + hFC transplant protocol was modified to add fludarabine conditioning and to perform the living donor transplants sequentially, with HSC + hFC administered one month prior to the kidney graft placement.

The first transplant (stage 1 FCRx) was performed in March 2008 (Patient #15 in Table 7). She is a 31-year-old female whose husband was her donor. The patient is currently in her nadir period and is doing well as an outpatient. A flow crossmatch performed on day 14 was negative (Table 8). In this assay, the binding of antibodies to donor T and B cells was measured by flow cytometric analysis in MCDF units.

**Table 8: Flow Crossmatch**

| | T cell | | | B cell | | |
|---|---|---|---|---|---|---|
| | % T cells | MCDF | Positive/ Negative | % B cells | MCDF | Positive/ Negative |
| Negative control | 57 | 250 | - | 5 | 301 | - |
| Positive control | 52 | 495 | + | 5 | 534 | + |
| Patient #15 | 54 | 245 | - | 5 | 246 | - |
| Patient #15 | 52 | 252 | - | 5 | 266 | - |

These results demonstrated the safety of the non-myeloablative conditioning and the feasibility of the HSC + hFC process and product in solid organ transplant. It is noted that none of the recipients became sensitized to the donor.

### Section E-Treatment of Thalassemia in Humans

Five individuals at high risk for morbidity and mortality from their thalassemia are enrolled on the protocol according to the inclusion and exclusion criteria below.

### Example 1-Inclusion criteria

The following criteria were established to identify individuals with thalassemia who have a high predicted morbidity and are at risk for early mortality: patients with alpha or beta thalassemia major; or patients with other complex and transfusion-dependent hemoglobinopathies. Individuals must also meet all of the following general inclusion criteria: individuals must have a related donor (identical or mismatched for 1, 2 or 3 HLA-A, -B or -DR loci); individuals must have adequate cardiopulmonary function as documented by echocardiogram or radionuclide scan (shortening fraction >26% or ejection fraction >40% or > 80% of normal value for age); individuals must have adequate pulmonary function documented by FEV of 50% of predicted for age and/or DLCO (corrected for hemoglobin) ≥50% of predicted for age for patients > 10 years of age (if patient cannot perform PFT's, resting pulse oximeter > 85% on room air or clearance by the pediatric or adult pulmonologist is required); individuals must have adequate hepatic function as demonstrated by a serum albumin > 3.0 mg/dL, and SGPT or SGOT < 5 times the upper limit of normal; and individuals must have adequate renal function as demonstrated by a serum creatinine < 2 mg/dL. If serum creatinine is > 2 mg/dL, then a creatinine clearance test or nuclear medicine GFR should document GFR of ≥ 50 ml/min/1.73 m2. There are no age limits for this protocol.

### Example 2-Exclusion criteria

Individuals are excluded from this trial if they meet any of the following criteria: the individual lacks related donors; the individual has uncontrolled infection or severe concomitant diseases, and may not tolerate reduced intensity transplantation; the individual exhibits severe impairment of functional performance as evidenced by a Karnofsky (patients >16 years old) or Lansky (children <16 years old) score of <70%; the individual exhibits renal insufficiency (GFR < 50 ml/min/1.73 m2); the individual has a positive human immunodeficiency virus (HIV) antibody test result; the individual is pregnant as indicated by a positive serum HCG test; the individual's only donor is pregnant at the time of intended transplant; the individual is of childbearing potential and is not practicing adequate contraception; the individual has been exposed to previous radiation therapy that would preclude TBI; the individual is a Jehovah's witness; the individual has uncontrolled hypersplenism; or the individual exhibits severe alloimmunization with inability to guarantee a supply of adequate PRBC donors.

### Example 3-Recipient evaluation

A complete history and physical examination of the individual is performed. Estimation of pre-HSCT Lansky or Karnofsky status is obtained. The history includes: age of diagnosis, overall growth and development, frequency and number of transfusions, any aplastic crises, prior treatment (e.g., Hydrea), baseline HbF plus A2 levels, alloimmunization status, treatment and dates, any MRI scans, transfusion therapy, infections, aseptic necrosis, history of hepatitis, iron overload, prior liver biopsies, and pathologic findings.

The following hematological tests are performed: CBC (Hgb, Hct, MCV, MCHC, RDW, platelet, white blood cell count), differential count, reticulocyte count, ferritin, folate, quantitative Hgb electrophoresis, PT, PTT, fibrinogen, direct and indirect Coombs test. In addition, the alpha gene number is determined, the beta-globin haplotype is determined, globin chain synthetic studies are performed, and the subject is ABO Rh typed and screened.

The following chemistries are obtained: total and direct bilirubin, SGPT,SGOT, alkaline phosphatase, Protein C, IgG subclasses, albumin, Ca++/PO4++/Mg++, serum electrolytes, BUN/creatinine, urinalysis, creatinine clearance/ GFR; and endocrine levels of T4, TSH, FSH, LH, and growth hormone.

The individual is HLA typed (HLA A, B, C, DQ and DR typing) based on molecular analysis.

The following diagnostic tests are performed on the individual: a CT scan (brain, sinuses, chest, abdomen, pelvis), PFTs (crying vital capacity for younger children unable to perform conventional PFT, DLCO for patients > 10 years), EKG, echocardiogram or MUGA scan, liver and spleen scan, ultrasound of gall bladder, bone age, and estradiol or testosterone.

The individual is screened for the following infectious markers: CMV, IgG, PCR, HSV & VZV IgGs, HIV 1 and 2 antibody and PCR, HTLV 1 and 2 antibody, Hepatitis B surface antigen, Hepatitis B core antibody, Hepatitis C antibody and PCR, EBV IgG and IgM, toxoplasma IgG and IgM, West Nile Virus NAT, *Trypanosoma cruzi* (Chagas) antibody, RPR or equivalent.

### Example 4-Donor evaluation and selection

HLA-identical donor and recipients are used, or donor and recipient mismatched pairs (e.g., up to haploidentical (parent, aunt, uncle, cousin, or sibling)) are used. Family members willing to donate bone marrow are HLA-typed. The best available match is selected. All donors participating are evaluated as per FDA regulations for donor screening prior to stem cell harvest. All evaluations are completed within 30 days of the transplant. Pediatric donors are considered for mobilization. If the donor is not a good candidate for apheresis, bone marrow is harvested from the iliac crest. If more than one related donor is available, the closer matching, younger, and/or CMV-negative donor is selected. All donors are placed on iron replacement therapy. Pheresed donors can be supplemented with Vitamin K and/or calcium.

Donors are screened as described herein and the following information is obtained. The history and physical examination of the donor is obtained including pregnancy and transfusion history. Donors are screened for CBC, differential; PT with INR, PTT and fibrinogen; ABO and Rh Type and screen, ferritin, iron and TIBC; HLA typing: HLA class I (-A, -B, -C) and class II (-DR, -DQ) typing by molecular analysis; hemoglobin electrophoresis (thalassemia trait is acceptable); SGPT or SGOT, alkaline phosphatase, and bilirubin (total and direct); serum pregnancy test; serum electrolytes, BUN, and creatinine; CMV, IgG, PCR, HSV & VZV IgG, HIV 1 and 2 antibodies and PCR, HTLV 1 and 2 antibodies, Hepatitis B surface antigen, Hepatitis B core antibody, Hepatitis C antibody and PCR, EBV IgG and IgM, Toxoplasma IgG and IgM, West Nile Virus NAT, *Trypanosoma cruzi* (Chagas), RPR or equivalent test; hepatitis B core antibody (if antibody-positive, perform PCR for viral DNA, accept donor if negative); hepatitis B surface antigen (reject Hepatitis B antigen positive donor); HCV antibody (positive donor is acceptable only if PCR for viral DNA is negative); Herpes Simplex Virus antibody (document status only; positive donor is not rejected); HIV I/II antibody (reject HIV I/II positive donor); HIV PCR (reject HIV PCR positive donor); HIV I/II antibody (reject HTLV I/II positive donor); CMV antibody titer (if positive and recipient is negative, consider another donor if available, otherwise CMV screening and prophylaxis is mandatory); serologic test for syphilis (if positive, perform a fluorescent treponemal antibody test; donor is accepted if fluorescent treponemal antibody is negative); chest X-ray, if the donor is greater than 21 years of age; and EKG if the donor is greater than 40 years of age.

### Example 5-Pre-transplantation treatment of donor

For donors, a total of 560 cc of blood will be collected for archiving of lymphocytes for immunocompetence testing. This can be obtained as a single blood donation pre-transplant (450 cc). The remaining eleven 10 cc-yellow top collection tubes are obtained eight weeks after the first donation. For pediatric bone marrow donors, no more than 3 ml/kg at any one time are drawn, and no more than 7 ml/kg over a six-week period are drawn as per the NIH guidelines for pediatric research blood draws.

Beginning day -4 (with respect to HSC + hFC infusion) and for up to +4 days, 10 µg/kg G-CSF is administered b.i.d. Collection begins on day -1. A minimum of 5 x 10⁶ CD34/kg total is collected. A maximum of two collections are done. With each blood stem cell donation, 5-10 ml of blood is taken at the start and at the end of the procedure to measure blood cell counts including enumeration of CD 34+ cells.

At two days and one week after donation, the donor is contacted to confirm whether any adverse events have occurred. The donor also is asked to donate a blood sample (7 µl) one month after donation to ensure blood counts have recovered. The donor is treated with therapeutic iron, Vitamin K or calcium as needed. The visits for G-CSF administration, blood stem cell donations, and blood draws are summarized below in Table 9 (e.g., an X marks what will occur on each visit).

**Table 9**

| Visits | Symptom Assessment | Filgrastim / G-CSF | Blood Stem Cell Donation | Blood Draws |
|---|---|---|---|---|
| Screening | X | | | X |
| Preparation, Day -3 | X | X | | X |
| Preparation, Day -2 | X | X | | |
| Preparation, Day -1 | X | X | | |
| Preparation, Day 0, First donation | X | X | X | X |
| Second donation* | X | | X | X |
| 2 days after donation | X | | | |
| 1 week after donation | X | | | |
| Potential blood draws to test for donor chimerism in the recipient (up to 3 years) | | | | X |

| | | | | |
|---|---|---|---|---|
| *2nd donation occurs only if sufficient cells are not obtained in the 1st collection | | | | |

### Example 6-Recipient conditioning

Individuals are examined by a radiation therapist to determine dosimetry for TBI. Central venous access is established in all patients prior to initiation of conditioning. Campath-1H is administered in a first session at day -53, -52, -51, and - 50 at a maximum dose of 30 mg and in a second session at a maximum dose of 20 mg administered at day -24, -23, -22, and -21. The pediatric dose of Campath is 10 mg/day on cycle one and 7 mg/day on cycle two. For smaller recipients and those less than one year of age, Campath-1H is dosed at a rounded up dose of 0.4 mg/kg for the first regimen, and at a rounded up dose of 0.3 mg/kg for the second regimen. The route of administration of the Campath, either subcutaneously or intravenously, is at the discretion of the attending physician. Start dates for Campath administration can be moved forward or backward 1-3 days to accommodate scheduling conflicts. Fludarabine is administered on day -5, -4, and -3. The individual receives TBI and begins cyclosporine immunosuppression at day -1. The second immunosuppressive medication, mycophenolate mofetil, is started the evening of HSC + hFC infusion (day 0). The conditioning regimen is shown in the following Table.

**Table 10. Conditioning Approach**

| | |
|---|---|
| Day -53 to - 50 | Campath-1H is administered at 30 mg/day for adults and 10 mg/day for children over each of the four days. |
| Day -24 to - 21 | Campath-1H is administered at 20 mg/day for adults and 7 mg/day for children over each of the four days. |
| Day -5 -4, - 3 | Fludarabine is administered at 30 mg/m2 intravenously over a period of 30 minutes on each of these three days. |
| Day -1 | Pre-transplant conditioning 200 cGy TBI (35-40 cGy/min); Cyclosporine is administered day -1 and continued until it has been determined that the patient has engrafted, or it has been demonstrated that the patient has failed to engraft, or at the discretion of the physician. If engraftment occurs, cyclosporine is continued for at least 12 months. If there is no engraftment, cyclosporine is discontinued. Marrow is processed to retain hFC and HSC using ferromagnetic approach. |
| Day 0 | HSC + hFC is administered. MMF is started. |

The radiation is delivered at day -1. The radiation dose is 200 cGy of 6 MV accelerator X-rays, delivered in one fraction. A dose rate of 35-40 cGy/minute is used, dependent on the distance, energy, and patient dimensions. Dose variations greater than 10% are evaluated and approved on an individual basis. Infusion of the HSCs + hFCs occurs on day 0. Patients receive daily penicillin or equivalent prophylaxis for 2 years post-transplant, or longer at the discretion of the treating physician.

### Example 7-HSC + hFC cell processing

The mobilized peripheral blood stem cells are incubated with monoclonal antibodies that are specific for alpha beta TCR T cells and B cells, then depleted by immunomagnetic separation. The composition of the infused cells is assessed by immunofluorescent staining for CD34 HSCs; CD8+/TCR-/CD56^{dim/neg} hFCs; γ8 T cells, and αβ-TCR+ T cells. The adequacy of cellular depletion is determined by flow cytometric analysis, and the clinician is notified of preliminary cell doses prior to infusion. The cell product also is analyzed for bacteria, fungus, and endotoxins. The HSC + hFC product is infused via a central venous line in a monitored setting per institutional guidelines.

The processed graft is administered to all subjects, and the graft is only limited based on the maximal allowable alpha beta TCR dose. However, only those subjects with a minimally acceptable graft (e.g., at least 5 x 10⁹ total leukocytes available from the collection to process; at least 5 x 10⁶ CD34/kg of recipient body weight; and a T cell depletion of less than 0.5 logs) are evaluated as described herein.

### Example 8-Cell dosing algorithm

As many HSCs, hFCs and progenitors as possible are administered within the context of a maximal allowable T cell dose to avoid GVHD. Presently, the maximum dosing is 3.0 x 10⁶ alpha beta T cells/kg recipient body weight. Recipients are followed for a minimum of 28 days. If engraftment is not observed, the maximal allowable alpha beta TCR dose is increased by one unit (4 x 10⁵/kg recipient body weight). The maximal allowable alpha beta TCR dose is increased until stable engraftment is achieved without significant GVHD. For HLA-matched transplants, there is no maximum T cell cap and cell dose does not increase based on the outcome of these matched transplants. For patients who are mismatched, the maximal allowable alpha beta TCR dose is determined.

**Table 11**

| | |
|---|---|
| HSC, hFC, progenitors | As many as possible |
| NK cells, B cells | Record and report doses |
| γ6-TCR+ T cells | Record and report doses |
| αβ-TCR+ T cells | For HLA matched, there will be no cap. For HLA mismatched, the maximal allowable will be determined by the last safe dose in the kidney, heart, liver tolerance, sickle cell, and MS protocols. |

If significant (>0.5%) donor engraftment is observed in the first 28 days, the individual is followed for an additional 28 days to assess the incidence of acute GVHD.

## Claims

1. A cellular composition comprising:
at least 30% human facilitating cells (hFCs) having a phenotype of CD8+ / alpha beta TCR- / delta gamma TCR- / CD56^{dim/neg}/ CD3+ / CD16+ / CD19+ / CD52+, further comprising hematopoietic stem cells (HSCs), wherein the HSCs have a phenotype of CD34+, wherein the HSCs are MHC-matched with the hFCs.

2. The cellular composition of claim 2 wherein the hFCs further have a phenotype of CXCR4+, CD123+, HLADR+, NKp30+, NKp44+, NKp46+, CD11c+, and CD162+.

3. The cellular composition of claim 2 wherein the hFCs further have a phenotype of CD11a+, CD11b+, CD62L+, and FoxP3+.

4. The cellular composition of claim 1, wherein the hFCs CD56^{dim/neg} phenotype is CD56^{dim}.

5. The cellular composition of claim 1, wherein the hFCs improve the engraftment ability of said HSCs compared to HSCs engrafted in the absence of said hFCs.

6. The cellular composition of claim 1 comprising at least about 50% of the hFCs.

7. The cellular composition of claim 6 comprising at least about 75% of the hFCs.

8. The cellular composition of claim 7 comprising at least about 90% of the hFCs.

9. The cellular composition of claim 1, wherein the phenotype of the cells is determined by antibody staining or flow cytometry.

10. A pharmaceutical composition comprising the cellular composition of claim 1.

11. The pharmaceutical composition of claim 10 for use in a method of treating a human suffering from a disease or for use in a method of transplanting donor cells, tissues, or organs into a human recipient, comprising
administering the composition to the human.

12. The composition for use in a method of treating a human according to claim 11 wherein the method further comprises:
partially conditioning the human by exposure to total body irradiation, an immunosuppressive agent, a cytoreduction agent, or combinations thereof prior to the administration of the pharmaceutical composition.

13. The composition for use in a method of treating a human according to claim 12 wherein the total body irradiation is 200 cGy.

14. The composition for use in a method of treating a human according to claim 11, wherein the pharmaceutical composition is administered intravenously.

15. The composition for use in a method of treating a human according to claim 11, wherein the disease is an autoimmune disease.

16. The composition for use in a method of treating a human according to claim 15 wherein the autoimmune disease is diabetes, multiple sclerosis, or systemic lupus erythematosus.

17. The composition for use in a method of treating a human according to claim 11 wherein the disease is an infection by an immunodeficiency virus or hepatitis.

18. The composition for use in a method of treating a human according to claim 11 wherein the disease is chosen from a hematopoietic malignancy, anemia, hemoglobinopathies, and an enzyme deficiency.

19. The composition for use in a method of treating a human according to claim 11 wherein the donor tissue or organ is heart, skin, liver, lung, heart and lung, kidney, pancreas, or an endocrine organ.

20. The composition for use in a method of treating a human according to claim 19 wherein the endocrine organ is a thyroid gland, parathyroid gland, a thymus, adrenal cortex, or adrenal medulla.

21. The composition for use in a method of treating a human according to claim 19 wherein the donor cells are pancreatic islet cells, neurons, or myocytes.

## Patentansprüche

1. Zelluläre Zusammensetzung, Folgendes umfassend:
mindestens 30% menschliche Erleichterungszellen (hFCs) die einen Phänotyp von CD8+/Alpha Beta TCR-/Delta Gamma TCR-/CD56^{dim/neg}/CD3+/CD16+/CD19+/CD52+ aufweisen, ferner umfassend hämatopoetische Stammzellen (HSCs), wobei die HSCs einen Phänotyp von CD34+ aufweisen, wobei die HSCs mit den hFCs MHC-gematcht sind.

2. Zelluläre Zusammensetzung nach Anspruch 1, wobei die hFCs ferner einen Phänotyp von CXCR4+, CD123+, HLADR+, NKp30+, NKp44+, NKp46+, CD11c+ und CD162+ aufweisen.

3. Zelluläre Zusammensetzung nach Anspruch 2, wobei die hFCs ferner einen Phänotyp von CD11a+, CD11b+, CD62L+ und FoxP3+ aufweisen.

4. Zelluläre Zusammensetzung nach Anspruch 1, wobei der Phänotyp der hFCs CD56^{dim/neg} CD56^{dim} ist.

5. Zelluläre Zusammensetzung nach Anspruch 1, wobei die hFCs die Transplantierkeit der HSCs im Vergleich zu in Abwesenheit der hFCs transplantierten HSCs verbessern.

6. Zelluläre Zusammensetzung nach Anspruch 1, mindestens ungefähr 50% der hFCs umfassend.

7. Zelluläre Zusammensetzung nach Anspruch 6, mindestens ungefähr 75% der hFCs umfassend.

8. Zelluläre Zusammensetzung nach Anspruch 7, mindestens ungefähr 90% der hFCs umfassend.

9. Zelluläre Zusammensetzung nach Anspruch 1, wobei der Phänotyp der Zellen durch Antikörper-Färbung oder Durchflusszytometrie bestimmt wird.

10. Pharmazeutische Zusammensetzung, die die zelluläre Zusammensetzung nach Anspruch 1 umfasst.

11. Pharmazeutische Zusammensetzung nach Anspruch 10 zur Verwendung in einem Verfahren zum Behandeln eines an einer Krankheit leidenden Menschen oder zur Verwendung in einem Verfahren zum Transplantieren von Spenderzellen, Geweben oder Organen in einen menschlichen Empfänger, umfassend
die Verabreichung der Zusammensetzung an den Menschen.

12. Zusammensetzung zur Verwendung in einem Verfahren zum Behandeln eines Menschen nach Anspruch 11, wobei das Verfahren ferner umfasst:
teilweises Konditionieren des Menschen durch Einwirkung einer Ganzkörperbestrahlung, eines Immunsuppressivums, eines Zytoreduktionsmittels oder einer Kombination davon, vor der Verabreichung der pharmazeutischen Zusammensetzung.

13. Zusammensetzung zur Verwendung in einem Verfahren zum Behandeln eines Menschen nach Anspruch 12, wobei die Ganzkörperbestrahlung 200 cGy beträgt.

14. Zusammensetzung zur Verwendung in einem Verfahren zum Behandeln eines Menschen nach Anspruch 11, wobei die pharmazeutische Zusammensetzung intravenös verabreicht wird.

15. Zusammensetzung zur Verwendung in einem Verfahren zum Behandeln eines Menschen nach Anspruch 11, wobei die Krankheit eine Autoimmunkrankheit ist.

16. Zusammensetzung zur Verwendung in einem Verfahren zum Behandeln eines Menschen nach Anspruch 15, wobei die Autoimmunkrankheit Diabetes, Multiple Sklerose oder systemischer Lupus erythematodes ist.

17. Zusammensetzung zur Verwendung in einem Verfahren zum Behandeln eines Menschen nach Anspruch 11, wobei die Krankheit eine Infektion durch einen Immunschwäche-Virus oder Hepatitis ist.

18. Zusammensetzung zur Verwendung in einem Verfahren zum Behandeln eines Menschen nach Anspruch 11, wobei die Krankheit aus einer hämatopoetischer Bösartigkeit, Anämie, Hämoglobinpathien und einem Enzymmangel ausgewählt ist.

19. Zusammensetzung zur Verwendung in einem Verfahren zum Behandeln eines Menschen nach Anspruch 11, wobei das Spendergewebe oder -organ ein Herz, Haut, Leber, Lunge, Herz und Lunge, Niere, Bauchspeicheldrüse oder ein endokrines Organ ist.

20. Zusammensetzung zur Verwendung in einem Verfahren zum Behandeln eines Menschen nach Anspruch 19, wobei das endokrine Organ eine Schilddrüse, Nebenschilddrüse, ein Thymus, eine Nebennierenrinde oder ein Nebennierenmark ist.

21. Zusammensetzung zur Verwendung in einem Verfahren zum Behandeln eines Menschen nach Anspruch 19, wobei die Spenderzellen Inselzellen der Bauchspeicheldrüse, Neuronen oder Myozyten sind.

## Revendications

1. Composition cellulaire comprenant :
au moins 30 % de cellules facilitantes humaines (hFC) ayant un phénotype de CD8+/alpha bêta TCR-/delta gamma TCR-/CD56^{dim/neg} CD3+/CD16+/CD19+/CD52+, comprenant en outre des cellules souches hématopoïétiques (HSC), les HSC ayant un phénotype de CD34+, les HSC étant CMH compatibles avec les hFC.

2. Composition cellulaire selon la revendication 2, dans laquelle les hFC ont en outre un phénotype de CXCR4+, CD123+, HLADR+, NKp30+, NKp44+, NKp46+, CD11c+ et CD162+.

3. Composition cellulaire selon la revendication 2, dans laquelle les hFC ont en outre un phénotype de CD11a+, CD11b+, CD62L+ et FoxP3+.

4. Composition cellulaire selon la revendication 1, dans laquelle le phénotype CD56^{dim/neg} des hFC est CD56^{dim}.

5. Composition cellulaire selon la revendication 1, dans laquelle les hFC améliorent l'aptitude au greffage desdits HSC par comparaison aux HSC greffés en l'absence desdits hFC.

6. Composition cellulaire selon la revendication 1, comprenant au moins environ 50 % des hFC.

7. Composition cellulaire selon la revendication 6, comprenant au moins environ 75 % des hFC.

8. Composition cellulaire selon la revendication 7, comprenant au moins environ 90 % des hFC.

9. Composition cellulaire selon la revendication 1, dans laquelle le phénotype des cellules est déterminé par un marquage par anticorps ou une cytométrie de flux.

10. Composition pharmaceutique comprenant la composition cellulaire selon la revendication 1.

11. Composition pharmaceutique selon la revendication 10 pour une utilisation dans une méthode de traitement d'un être humain souffrant d'une maladie ou pour une utilisation dans une méthode de transplantation de cellules, de tissus ou d'organes de donneur dans un receveur humain, comprenant
l'administration de la composition à l'être humain.

12. Composition pour une utilisation dans une méthode de traitement d'un être humain, selon la revendication 11, dans laquelle la méthode comprend en outre :
le conditionnement partiel de l'être humain par exposition à une irradiation corporelle totale, un agent immunosuppresseur, un agent de cytoréduction ou des combinaisons de ceux-ci avant l'administration de la composition pharmaceutique.

13. Composition pour une utilisation dans une méthode de traitement d'un être humain, selon la revendication 12, dans laquelle l'irradiation corporelle totale est de 200 cGy.

14. Composition pour une utilisation dans une méthode de traitement d'un être humain, selon la revendication 11, dans laquelle la composition pharmaceutique est administrée par voie intraveineuse.

15. Composition pour une utilisation dans une méthode de traitement d'un être humain, selon la revendication 11, dans laquelle la maladie est une maladie auto-immune.

16. Composition pour une utilisation dans une méthode de traitement d'un être humain, selon la revendication 15, dans laquelle la maladie auto-immune est le diabète, la sclérose en plaques ou le lupus érythémateux disséminé.

17. Composition pour une utilisation dans une méthode de traitement d'un être humain, selon la revendication 11, dans laquelle la maladie est une infection par un virus de l'immunodéficience ou l'hépatite.

18. Composition pour une utilisation dans une méthode de traitement d'un être humain, selon la revendication 11, dans laquelle la maladie est choisie parmi une malignité hématopoïétique, l'anémie, les hémoglobinopathies et une déficience enzymatique.

19. Composition pour une utilisation dans une méthode de traitement d'un être humain, selon la revendication 11, dans laquelle le tissu ou l'organe de donneur est le coeur, la peau, le foie, le poumon, le coeur et le poumon, le rein, le pancréas ou un organe endocrine.

20. Composition pour une utilisation dans une méthode de traitement d'un être humain, selon la revendication 19, dans laquelle l'organe endocrine est une glande thyroïde, une glande parathyroïde, un thymus, le cortex surrénal ou la médullosurrénale.

21. Composition pour une utilisation dans une méthode de traitement d'un être humain, selon la revendication 19, dans laquelle les cellules de donneur sont des cellules des îlots pancréatiques, des neurones ou des myocytes.
